# EUROPÄISCHE PATENTANMELDUNG

(11) **EP 1 702 649 A1**
(43) Veröffentlichungstag der Anmeldung: **20.09.2006**
(21) Anmeldenummer: 06004299.1
(22) Anmeldetag: 03.03.2006
(51) Int. Cl.: A61N 1/39, A61B 5/0205, A61M 16/06

(54) **Modulares medizintechnisches Gerät**

(30) Priorität: 15.03.2005 DE 102005012200; 12.07.2005 DE 102005032431
(71) Anmelder: Weinmann Geräte für Medizin GmbH & Co. KG, 22525 Hamburg (DE)
(72) Erfinder: Wedler, Wolfgang, 21147 Hamburg (DE); Hofbauer, Eric, 20359 Hamburg (DE); Bolz, Armin, Prof., 91054 Buckenhof (DE)
(74) Vertreter: Klickow, Hans-Henning

(57) **Zusammenfassung**

Die Erfindung betrifft ein medizintechnisches Gerät zum Monitoring und/oder zur Therapie, mit einer Basisstation und wenigstens einem Modul. Das erfindungsgemäße medizintechnische Gerät ist dadurch gekennzeichnet, dass das wenigstens ein Modul im von der Basisstation getrennten Zustand funktionsfähig ist.

## Beschreibung

Die Erfindung betrifft ein modulares medizintechnisches Gerät zum Monitoring und/oder zur Therapie, mit einer Basisstation und wenigstens einem Modul.

Aus dem Stand der Technik bekannte medizintechnische Geräte weisen verschiedene Schwachpunkte auf. Beispielsweise stellen bei bekannten Monitoren von Defibrillatoren die mechanischen Komponenten die empfindlichsten Teile dar. Besonders kritisch sind der Drucker und der Patientenkartenleser mit der jeweiligen Feinmechanik sowie das elektromechanische Interface zum externen Speichermedium. Es erfordert einen beträchtlichen Entwicklung- und Kostenaufwand, diese Komponenten vor Flüssigkeiten (IPX 4), Staub, Vibrationen und Stößen (Falltest, 10g sind in jeden Fall zu erfüllen) schützen. Die erforderlichen konstruktiven Maßnahmen erhöhen das Gerätegewicht und vergrößern das Gehäusevolumen.

Zum anderen haben bekannte Monitore von Defibrillatoren für den Rettungsdienst den Schwachpunkt, dass der Monitor und das Therapiegerät in ein Gerät integriert sind. Für Fälle, in denen nur ein Monitoring erforderlich ist, z.B. beim Patiententransport im Treppenhaus, Patient ansprechbar, würde ein Basismonitoring genügen. Trotzdem muss für einen Patiententransport in einem Fahrzeug das komplette Gerät zum Beispiel an der Trage bzw. am Patienten selbst befestigt werden oder von einer weiteren Person getragen werden. Durch begrenzte Kabellängen ist dies jedoch schwierig. In der Praxis wird der Patient daher während des Transports oft nicht überwacht.

Weitere Schwachpunkte sind:
- Das Gewicht und die Baugröße erschweren die Handhabung als mobile Einheit,
- durch zahlreiche Funktionsmodule wird die Bedienung vor Ort komplex und fehleranfällig, und
- die zahlreichen Funktionsmodule belasten die mobile Energieversorgung und reduzieren damit die Einsatzzeit außerhalb des Fahrzeuges.

Generell werden medizintechnische Geräte derzeit häufig derart konstruiert, daß äußerst komplexe und leistungsfähige Geräte bereitgestellt werden, die mit einer Vielzahl von Funktionen sowie Funktionselementen versehen sind. Bei der überwiegenden Anzahl aller Verwendungen sind die überwiegende Anzahl dieser Funktionen und Funktionselemente aber nicht erforderlich. Es wird dem Patienten bzw. dem sonstigen Anwender somit in der Regel ein Gerät bereitgestellt, das eine Leistungsfähigkeit aufweist, die deutlich oberhalb des jeweiligen Anwendungsprofils liegt und die einen entsprechen hohen Gerätepreis und letztlich eine hohe Störungsanfälligkeit zur Folge hat.

Es ist daher Aufgabe der Erfindung, ein medizintechnisches Gerät bereitzustellen, das in der Handhabung vereinfacht und komfortabler ist.

Diese Aufgabe der Erfindung wird dadurch gelöst, daß das wenigstens eine Modul im von der Basisstation getrennten Zustand funktionsfähig ist.

Durch die erfindungsgemäße Konstruktion ist es möglich, ein einzelnes Funktionsmodul derart auszubilden, daß alle elementaren Funktionen bereitgestellt werden, die zumindest bei der überwiegenden Anzahl aller Anwendungen erforderlich sind. Im Bereich der Basisstation werden hingegen diejenigen Funktionen implementiert, die nur selten oder nur für Spezialanwendungen erforderlich sind, so daß es insbesondere möglich ist, eine Vielzahl von Funktionsmodulen einer einzelnen Basisstation zuzuordnen und die Basisstation zeitlich nacheinander mit mehreren der Funktionsmodule zu kombinieren. Darüber hinaus ist es auch möglich, die Basisstation konfigurierbar auszubilden, so daß bei einer Kombination der Basisstation mit einem bestimmten Funktionsmodul zusätzlich auch noch Zusatzmodule implementiert werden können, um weitere Funktionen bereitzustellen.

Gemäß einer weiteren Ausführungsform ist es auch möglich, einzelne Funktionsmodule ohne Kombination mit der Basisstation mit Zusatzmodulen zu kombinieren, um eine individuell konfigurierbare Leistungsfähigkeit bereitzustellen. Auch hierdurch können aufbauend auf einem modularen Gerätekonzept unter Verwendung einer Vielzahl in großen Stückzahlen produzierter Funktionsmodule sowie Zusatzmodul individuell konfigurierte Anwendungsanforderungen erfüllt werden.

Im Fall eines Patiententransports muß beispielsweise nur das Modul mit dem Patienten mitgeführt werden, jedoch nicht das komplette schwere Gerät. Dabei beträgt die Gewichtsreduktion bei einem Defibrillator 10 %. Dies entspricht ca. 750 Gramm vom Gerätegewicht (Drucker ca. 400 Gramm, Patientenkartenleser ca. 150 Gramm, GSM-Modul ca. 100 Gramm, Schnittstelle für externe Speichermedien ca. 100 Gramm). Ferner wird so eine Reduzierung des Gehäusevolumens um ca. 1,5 Liter erreicht (Drucker ca. 0,8 Liter, GSM-Modul, Patientenkartenleser ca. 0,3 Liter und Schnittstelle für externe Speichermedien ca. 0,4 Liter). Vorteilhaft ist außerdem, dass die ausgelagerten Module keine Energie aus einer mobilen Energieversorgung benötigen. Dies erhöht die Einsatzdauer des Gerätes.

Vorzugsweise weist die Basisstation wenigstens eine Anzeigeeinrichtung auf, die beispielsweise ein Display wie ein Touchscreen sein kann und eine verbesserte Übersichtlichkeit und einen erhöhten Einstellkomfort bietet. Der Touchscreen dient als Eingabe- und Ausgabeeinheit bzw. als Benutzerinterface, über das das medizintechnische Gerät bedient werden kann.

In einer bevorzugten Ausführungsform ist vorgesehen, dass die Basisstation zum Datenaustausch mit dem wenigstens einem Modul ausgebildet ist. So können Daten, beispielsweise Messwerte aus der Patientenüberwachung, zur Basisstation übertragen und auf dem Display angezeigt werden und umgekehrt Einstellparameter auf dem Display visualisiert werden und von der Basisstation zum Modul übertragen werden. Dies erlaubt es, eine Basisstation für eine Vielzahl von Modulen bereitzuhalten, wobei sich eine Kostenersparnis dadurch ergibt, dass nicht jedes Modul mit einem Display ausgestattet sein muss.

Dabei ist vorzugsweise vorgesehen, dass das Modul und die Basisstation zum drahtlosen und/oder drahtgebundenen und/oder optischen Datenaustausch ausgebildet sind. Hierbei ist das Basismodul als Master ausgebildet und hält notwendige Informationen, wie beispielsweise Patientendaten und individuelle Einstellungen, bereit. Hierzu gehören auch ein Kennungscode des jeweiligen Moduls sowie ein patientenindividueller Code, die wie die Behandlungshistorie übertragen werden. Aktuelle Meßwerte werden im Funktionsmodul gesammelt.

Die drahtlose Datenübertragung erfolgt vorzugsweise mittels Internet, Bluetooth, Funk, SMS, Global System for Mobile Communications (GSM), Enhanced Data Rates for GSM Evolution (EDGE), Wide Band Code Division Multiple Access (WCDMA), optisch, ZigBee, WLAN oder Infrarot. So kann auch ein Datenaustausch erfolgen, wenn sowohl das Modul und die Basisstation sich in einem Radius von bis 100 Metern befinden. Alternativ kann die Möglichkeit vorgesehen sein, eine verbindung mit einem Mobiltelefon herzustellen, damit Daten über größere Entfernungen übermittelt werden können. Übermittelte Daten können sein: Kalibrierwerte, Messbereiche, Extremwerte, Druck, Flow, Beatmungsparameter, Beatmungshistorie, voreingestellte Warn-/Alarmbereiche, insbesondere bei Erreichen der Alarmbereiche, Zustandsdaten des Patienten und/oder des Gerätes sowie eine Gerätekennung. Außerdem kann die Möglichkeit vorgesehen sein, Daten drahtgebunden oder drahtlos an einen PC zu übertragen.

Des weiteren kann vorgesehen sein, sowohl das Basisgerät als auch das wenigstens eine Modul mit RFID (radio frequency identification) zu versehen, so dass bei Annäherung eines Modul an das Basisgerät durch eine entsprechende programmtechnische Einrichtung bewirkt wird, dass die Daten des Moduls mit einer Liste von Kennungen verglichen werden und bei Vorliegen einer bekannten Kennung die Basisstation sofort gespeicherte Moduldaten und/oder Patientendaten aufruft und anzeigt.

Außerdem ist es möglich, die Einrichtungen zum Datenaustausch derart weiterzubilden, dass im Rahmen einer Fernwartung Softwareupdates installiert werden können.

Vorzugsweise ist vorgesehen, dass die Basisstation eine Aufnahme für das wenigstens ein Modul aufweist. Die Aufnahme kann als Schubfach mit einem Rastmechanismus ausgebildet sein, die einen sicheren Halt des Moduls in der Aufnahme gewährleistet und zugleich eine problemlose Entnahme im Bedarfsfall ermöglicht.

In einer bevorzugten Weiterbildung ist vorgesehen, dass die Basisstation und das wenigstens eine Modul komplementär zueinander ausgebildete Ladekontakte aufweisen, sodass ein in die Aufnahme eingeschobenes Modul über die Ladekontakte mit elektrischer Energie versorgt wird und zugleich zusätzlich eine Verbindung zum Austausch von Daten gegeben ist.

In einer bevorzugten Ausführungsform ist vorgesehen, dass die Basisstation eine Alarmeinrichtung aufweist. Die Alarmeinrichtung ist derart ausgebildet, dass auf empfangene Zugangsdaten von wenigstens einem Modul ein optisches, akustisches, mechanisches, elektrisches oder pneumatisches Signal erzeugt wird.

Vorzugsweise ist vorgesehen, dass die Basisstation tragbar ausgebildet ist, sodass das medizintechnische Gerät auch z.B. in Rettungsfahrzeugen verwendet werden kann.

Dabei ist bevorzugt vorgesehen, dass sowohl die Basisstation als auch das wenigstens eine Modul je eine Einrichtung zur netzunabhängigen Energieversorgung aufweisen. Hierzu sind Akkus, Solarzellen oder Brennstoffzellen neben einem Netzanschluss und einem Ladegerät vorgesehen.

In einer weiteren Ausführungsform ist vorgesehen, dass ein Funktionsmodul als Therapiegerät ausgebildet ist. Hierbei werden unter Therapiegeräten beispielsweise Beatmungsgeräte, Absaugpumpen, Sauerstoffgeräte oder Defibrillatoren oder andere medizintechnische Geräte verstanden.

Vorzugsweise umfasst das erfindungsgemäße medizintechnische Gerät weiter eine Dockingstation zur Aufnahme wenigstens einer Basisstation. Die Dockingstation ist zum drahtgebundenen und/oder drahtlosen Datenaustausch mit dem Basisgerät ausgebildet und kann dabei eine Vielzahl technischer Komponenten aufweisen, z.B. eine Ladeschnittstelle, einen Drukker, einen Patientenkartenleser, ein GSM-Modul, externe Speichermedien, eine Tasche für Zubehör, einen Pacer, erweiterte Sensorik für Intensivtransporte (IPB, CO₂, Temperatur) sowie eine Schnittstelle zu einem externen Monitor. Dabei kann die Dockingstation zum Einbau in ein Rettungsfahrzeug wie ein Kfz oder einen Hubschrauber ausgebildet sein. Alternativ kann die Dockingstation auch zum Einsatz in Gebäuden, z.B. Krankenhäusern, ausgebildet sein und zugleich einen Wandhalter aufweisen. Die Dockingstation kann auch neben der Basisstation stehen.

In einer bevorzugten Ausführungsform ist vorgesehen, dass die Dockingstation und das Basisgerät komplementär zueinander ausgebildete Ladekontakte aufweisen, sodass das Basisgerät durch die Dockingstation mit elektrischer Energie versorgt wird. Eine elektrische Verbindung wird hergestellt, indem das Basisgerät in eine Aufnahme der Docking-station eingesetzt wird, wobei es nicht erforderlich ist, Verbindungen mit Leitungen und Steckern herzustellen.

Ferner gehören zur Erfindung die oben beschriebene Basisstation, das oben beschriebene Modul sowie die oben beschriebene Dockingstation.

In den Zeichnungen sind Ausführungsbeispiele der Erfindung dargestellt. Es zeigen:
- Fig. 1: eine schematische Darstellung eines Basisgerätes mit Funktionsmodul,
- Fig. 2: ein Funktionsschema,
- Fig. 3 - 40: verschiedene Ausführungsbeispiele des Basisgerätes mit Funktionsmodul,
- Fig. 41: ein weiteres Ausführungsbeispiel der Erfindung,
- Fig. 42: ein Ablaufschema und
- Fig. 43: ein Diagramm zur Veranschaulichung des modularen Gerätesystems.

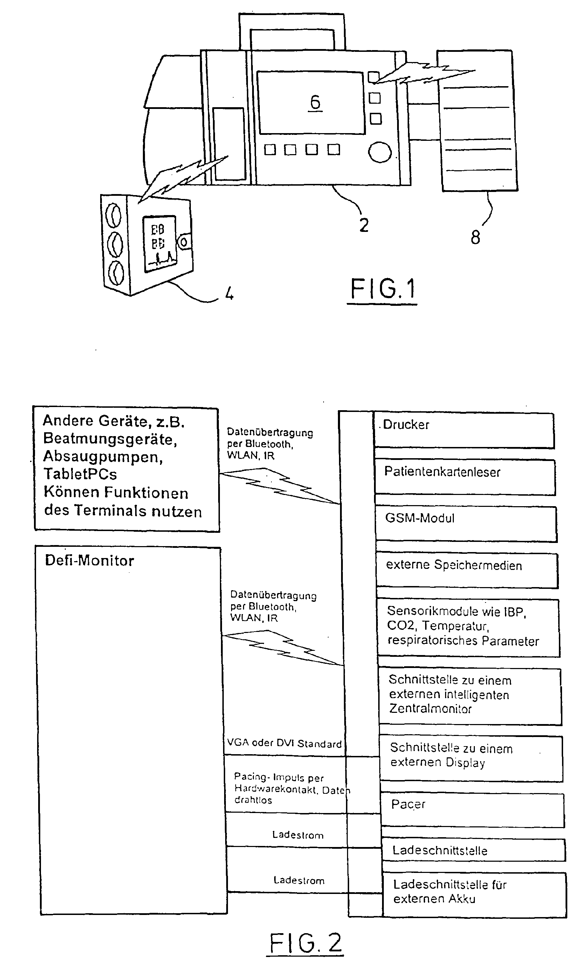

In Fig. 1 ist ein medizintechnisches Gerät für einen Einsatz in einem Rettungsfahrzeug dargestellt. Das medizintechnische Gerät umfasst eine Docking-Station (8) in dem Fahrzeug, ein Basisgerät (2) sowie eine Koppelbox (4), die über eine drahtlose Datenübertragung kommunizieren.

Die Docking-Station (2) im Fahrzeug umfasst alle Funktionsmodule, die nicht für einen Einsatz außerhalb des Fahrzeugs benötigt werden. Daher umfasst die Dockingstation (2) eine Ladeschnittstelle, einen Drucker (z.B. DIN A4 oder mit 50 bis 100 mm Druckbreite), einen Patientenkartenleser, ein GSM-Modul, externe Speichermedien, eine Tasche für Zubehör, einen Pacer, erweiterte Sensorik für Intensivtransporte (IPB, CO₂, Temperatur, respiratorisches Monitoring), Schnittstellen zu externen Monitoren und/oder Zentralmonitoren sowie eine Ladeschnittstelle, beispielsweise mit einem Ladefach für einen weiteren Akku. Dabei kann die Dokkingstation (8) programmtechnisch derart ausgebildet sein, dass diese in der Dockingstation (8) zusammengefassten Funktionsmodule von anderen Komponenten mitbenutzt werden können.

Das Basisgerät (2) weist eine als Touchscreen ausgebildete Anzeige (6) auf, mit dem Parameter des Basisgeräts (2) sowie Parameter einer Koppelbox (4), die mit dem Basisgerät (2) in Verbindung steht, verändert werden können. Dabei arbeitet das Basisgerät (2) im AED Modus und ist ferner für einen manuellen Defibrillations-Modus sowie zum drahtlosen Monitoring ausgebildet, während die Koppelbox (4) zum drahtlosen Monitoring mit EKG (z.B. 3-12 Kanal EKG), Sp0₂ und NIBP ausgebildet ist und damit ein Basismonitoring ermöglicht. Alternativ kann die Koppelbox (4) auch die Werte CO₂, IBP, Temperatur und andere Patientenparameter aufnehmen.

Die Koppelbox (4) weist ein kleines Display sowie Bedienelemente auf, wobei auf dem Display Patientenparameter numerisch und/oder graphisch dargestellt werden. Zusätzlich werden die Daten zum Basisgerät (2) drahtlos übertragen. Die Datenübertragung erfolgt dabei über Bluetooth, ZigBee, WLAN oder Infrarot. Die Reichweite beträgt bis zu 100 Meter.

Als elektrische Kontakte sind lediglich die Ladekontakte und die Kontakte zu einem Pacer-Modul beziehungsweise die Kontakte für das Signal zum externen Display ausgebildet. Zum Zwecke der Redundanz ist aber auch vorgesehen, eine Datenübertragung über Leitungen durchzuführen.

Es ist vorgesehen, dass während des Einsatzes die Bestätigungstaste des Druckers deaktiviert ist und die zu druckenden Daten abgespeichert werden. Erst wenn das Gerät wieder in der Nähe der Dockingstation ist, wird der Drucker veranlasst, alle Aufträge ausdrucken. Dies hat den Vorteil, dass eine Dokumentation zu jedem Zeitpunkt möglich ist, jedoch kein Papier anfällt.

Die Patientendaten werden jedoch erst im Fahrzeug selbst eingelesen, während die Telemetrie mit dem GSM-Modul aktiviert wird, sobald sich der Patient im Rettungsfahrzeug befindet.

Die Einsatzdaten des Gerätes hingegen werden automatisch auf einen externen Datenträger übertragen, sobald sich das Gerät im Wagen befindet.

Der Pacer wird selten verwendet (ca. 2 Prozent der Einsätze). Wichtig ist der Pacer zur Stabilisierung eines Patienten während des Transports.

Zum Zwecke einer Intensivverlegung wird ein Patient erst im Fahrzeug an die zusätzlich Monitoringmodule angeschlossen.

Sobald das Gerät am Terminal angedockt ist, werden die Monitoringdaten am externen Zentralmonitor dargestellt. Die Monitoring- und Benutzerdaten werden dabei drahtlos zu dem Zentralmonitor übertragen, aufgearbeitet und dargestellt.

wird das Gerät an das Terminal angedockt, wird das Gerät automatisch geladen.

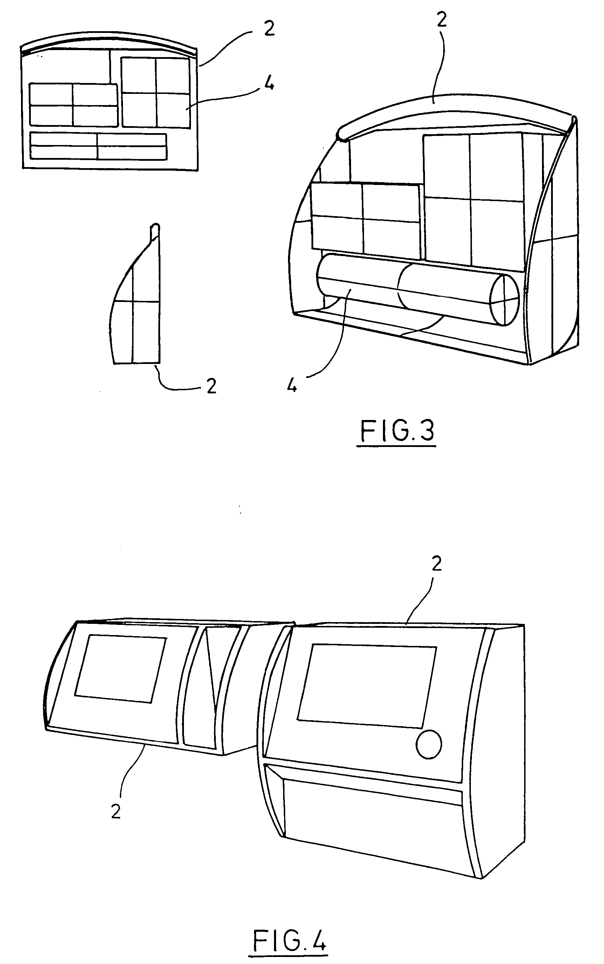

Die Fig. 3 und 4 zeigen als Halterungen ausgebildete Basisstationen (2) sowie in schematischer Darstellung Module (4).

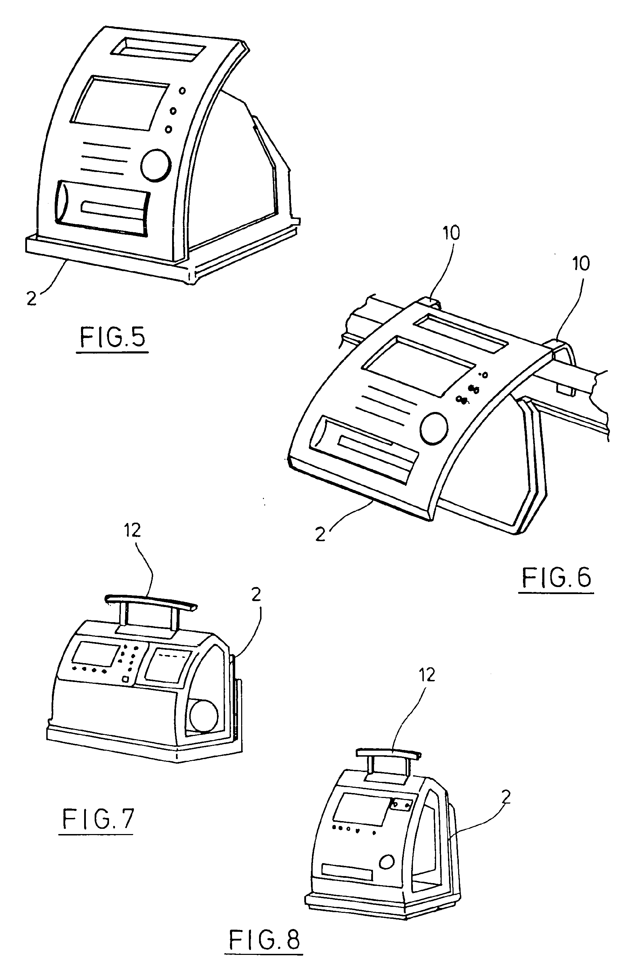

Die Fig. 5 bis 8 zeigen eine mit Haken (10) an einem Krankenhausbett befestigte Basisstation (2) sowie eine mit einem Handgriff (12) tragbar ausgebildete Basisstation (2), die zur Energieversorgung mit Akkus und einem Ladegerät versehen ist.

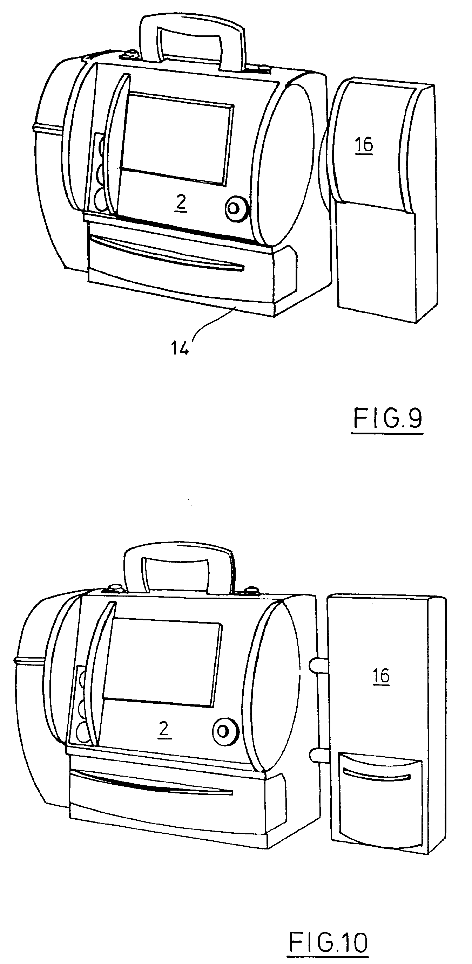

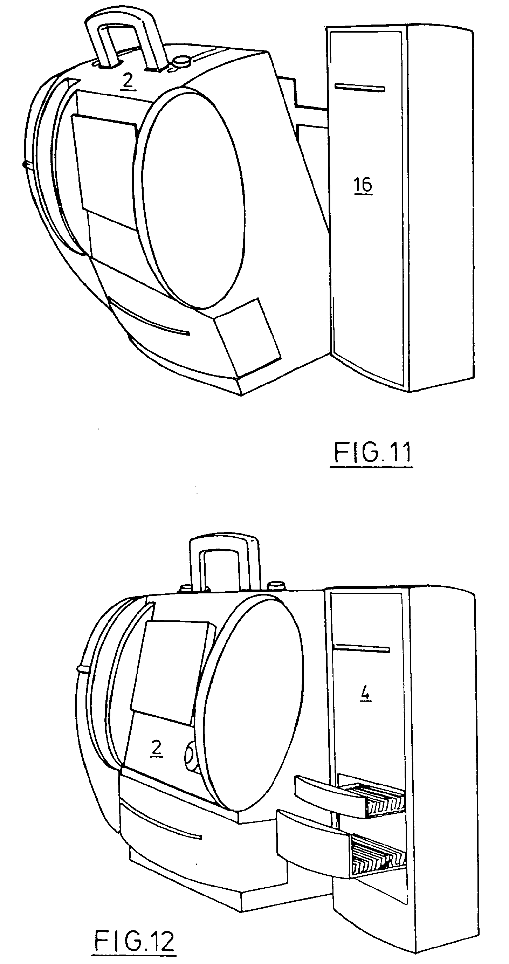

Die Fig. 9 bis 11 zeigen eine an einer wandhalterung (14) befestigte Basisstation (2) mit einem seitlich angeordneten Drucker, wobei anhand von Fig. 12 zu erkennen ist, dass das Basisgerät (2) durch seitliches Verschwenken eingefügt wird. Um nach einem Einsatz das Aufladen der Akkus zu ermöglichen, weist die Wandhalterung (14) Ladekontakte auf (nicht dargestellt), die zu Ladekontakten des Basisgeräts (2) korrespondieren und ein Aufladen ermöglichen.

Fig. 12 zeigt eine alternative Ausführungsform eines Moduls (4) für zubehör mit Schubfächern für eine Basisstation (4).

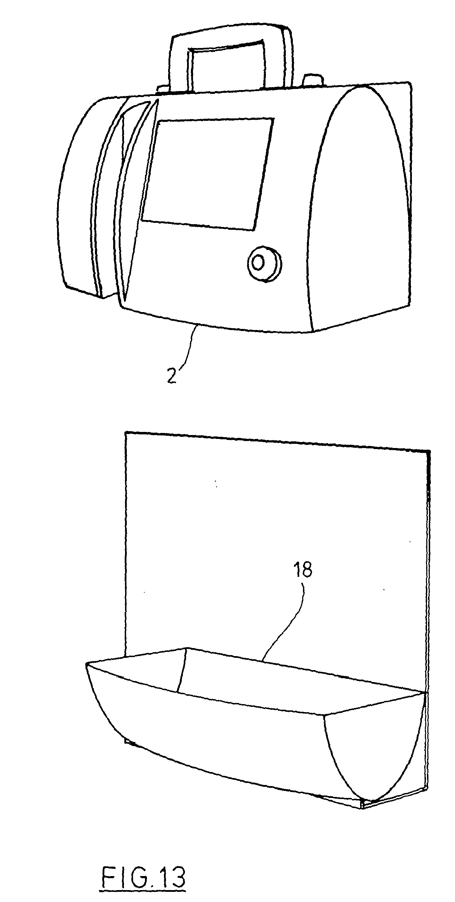

Fig. 13 zeigt eine schalenförmig ausgebildete Wandhalterung (18) zur Aufnahme einer Basisstation (2), die ebenfalls nicht dargestellte Ladekontakte aufweist.

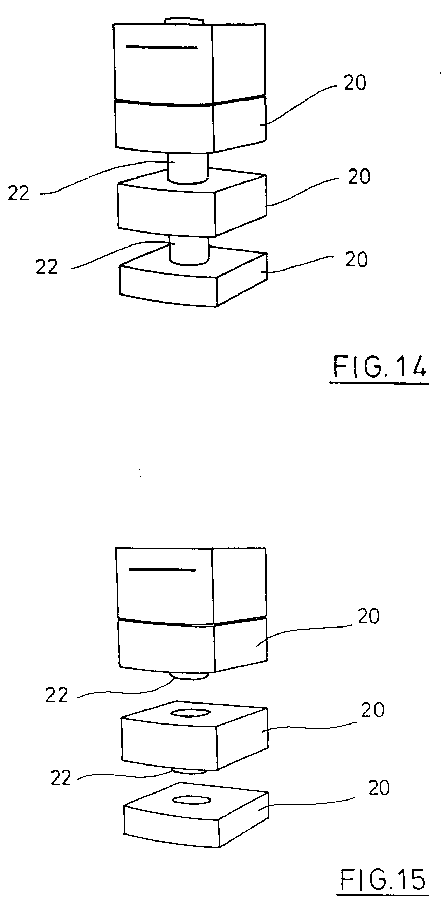

Die Fig. 14 und 15 zeigen eine weitere Ausführungsform, bei der mehrere Gerätemodule (20) verschiedener Funktion, beispielsweise Therapie, Anzeige (Display), Monitoring, Bedienung, Kommunikation, übereinander angeordnet sind und über zentral angeordnete und identisch ausgebildete Steckverbindungen (22) miteinander kommunizieren.

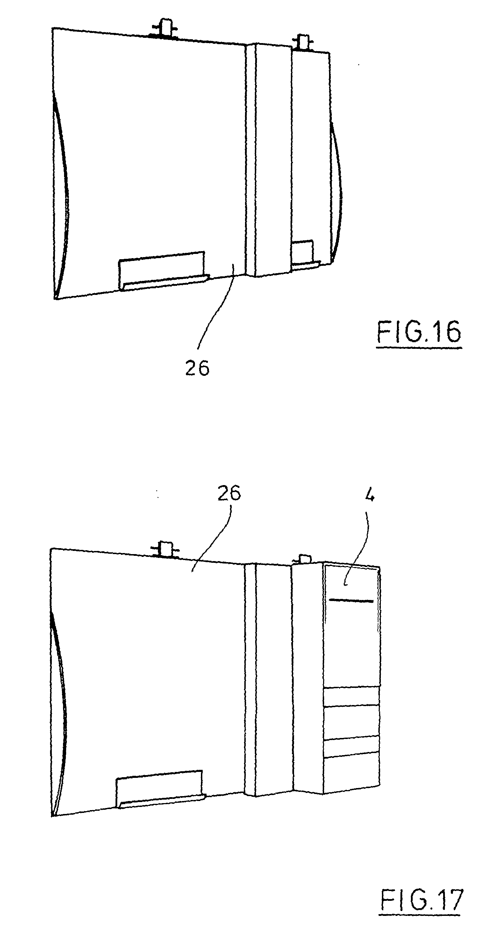

Die Fig. 16 und 17 zeigen verschiedene Ausführungsformen einer Wandhalterung (26) für eine Basisstation (2) mit und ohne Modul (4).

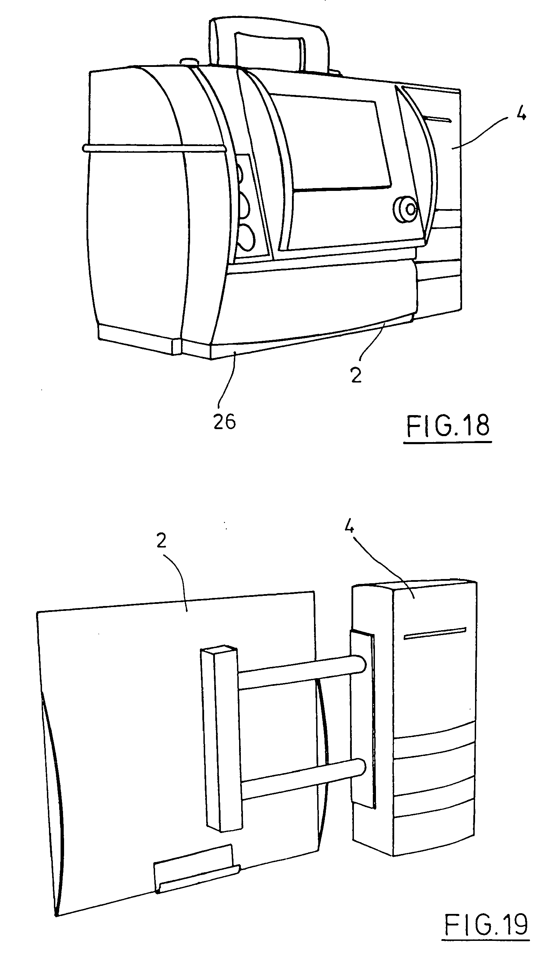

Die Fig. 18 zeigt die Wandhalterung der Fig. 16 und 17 mit einem Basisgerät.

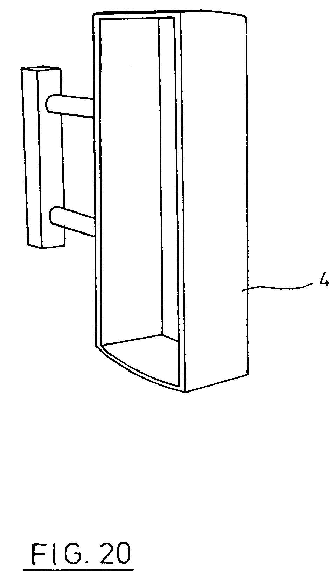

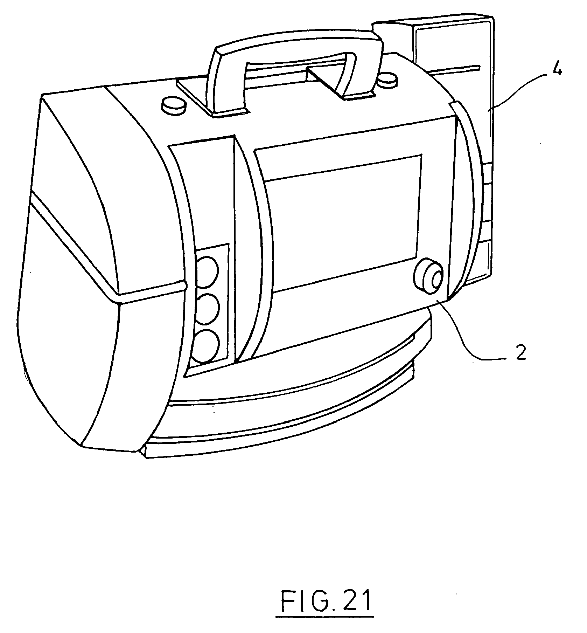

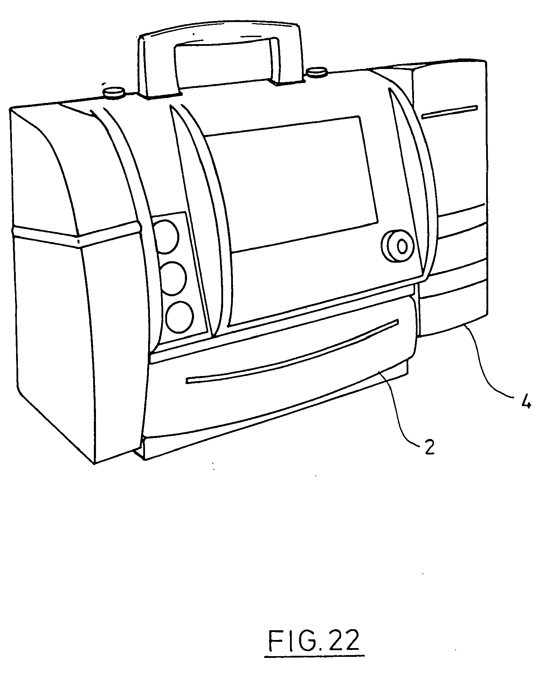

Die Fig. 19 bis 22 zeigen eine Basisstation (2) mit einem schwenkbaren befestigten Modul (4).

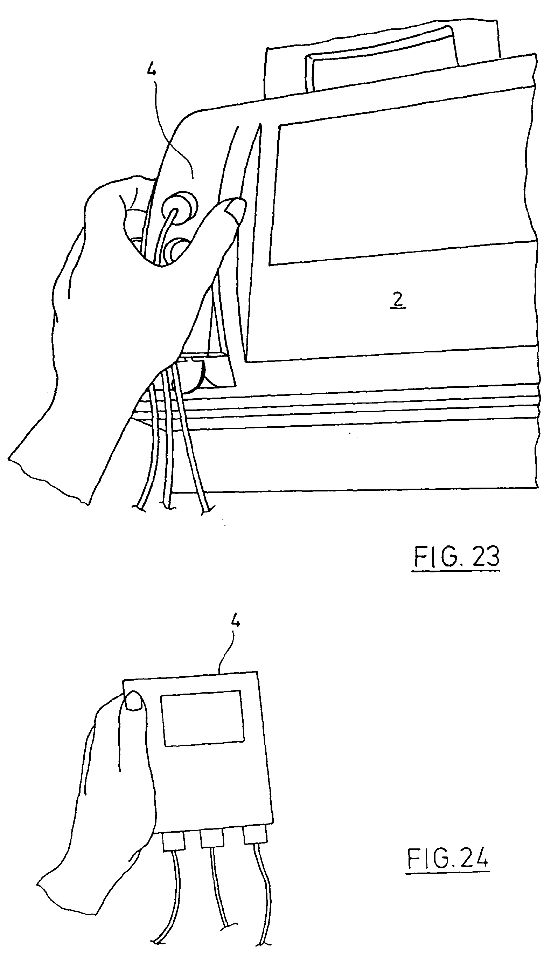

Die Fig. 23 bis 24 zeigen ein Zusatzmodul, dass als Koppelbox (4) ausgebildet ist und als Zusatz-Modul in ein Basisgerät (2) einsteckbar ist. Dabei weist das Zusatz-Modul ein eigenes Display (28) auf, wobei Bedienelemente (30) nicht mittig angeordnet sind, so dass das Zusatzmodul mit einer Hand gehalten und mit einer weiteren Hand bedient werden kann.

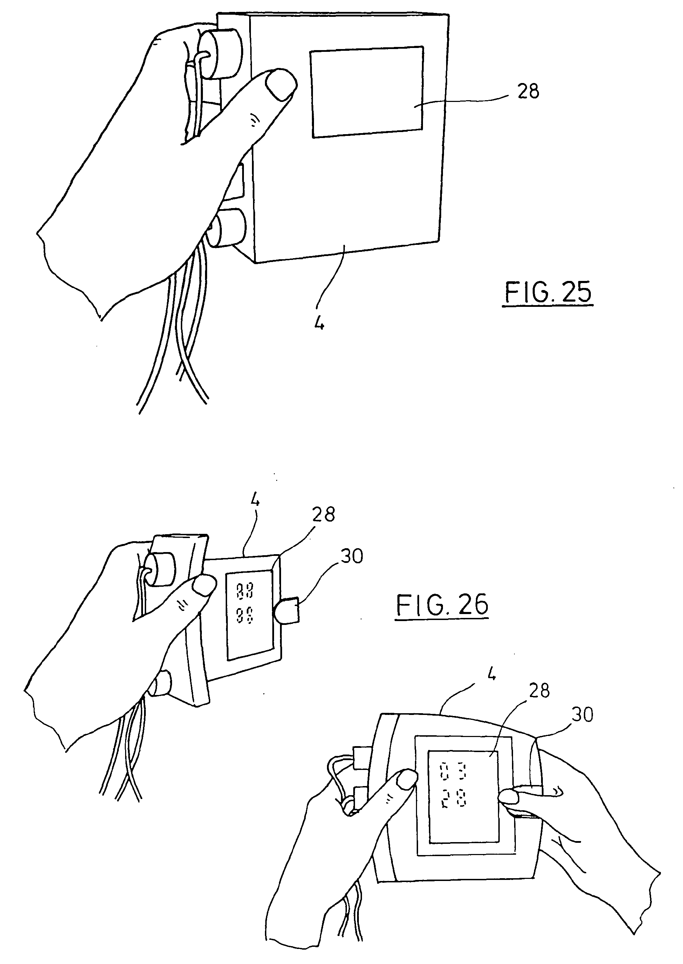

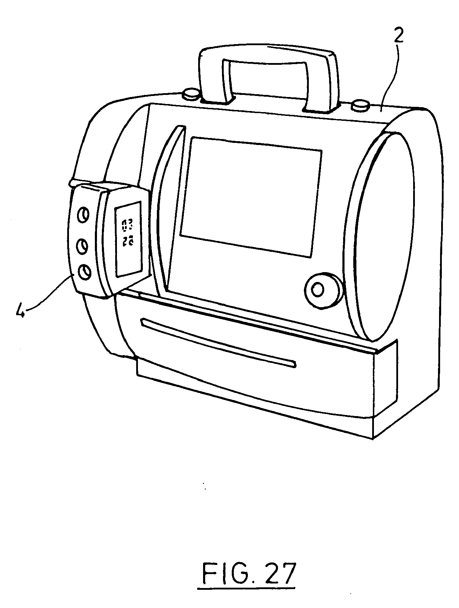

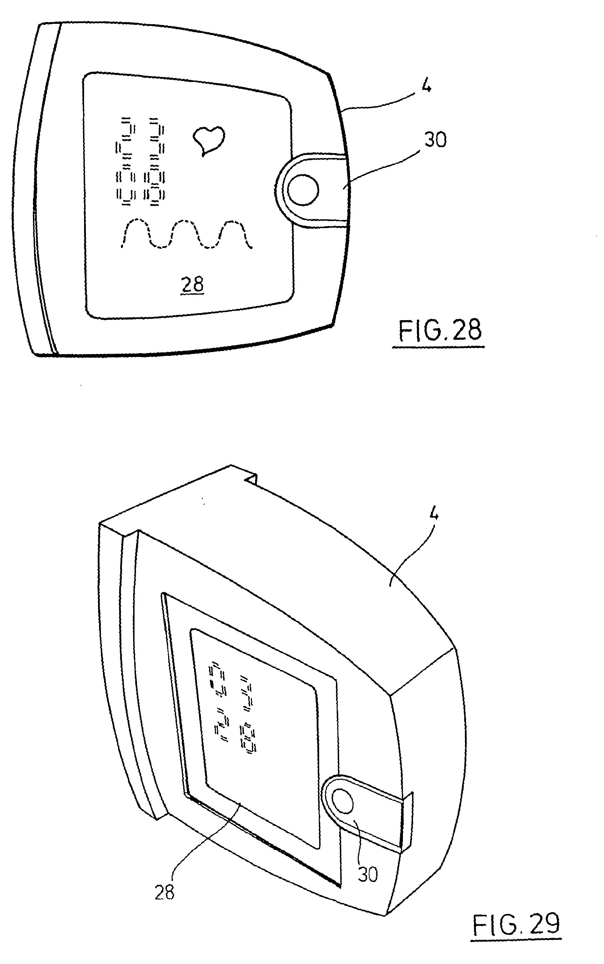

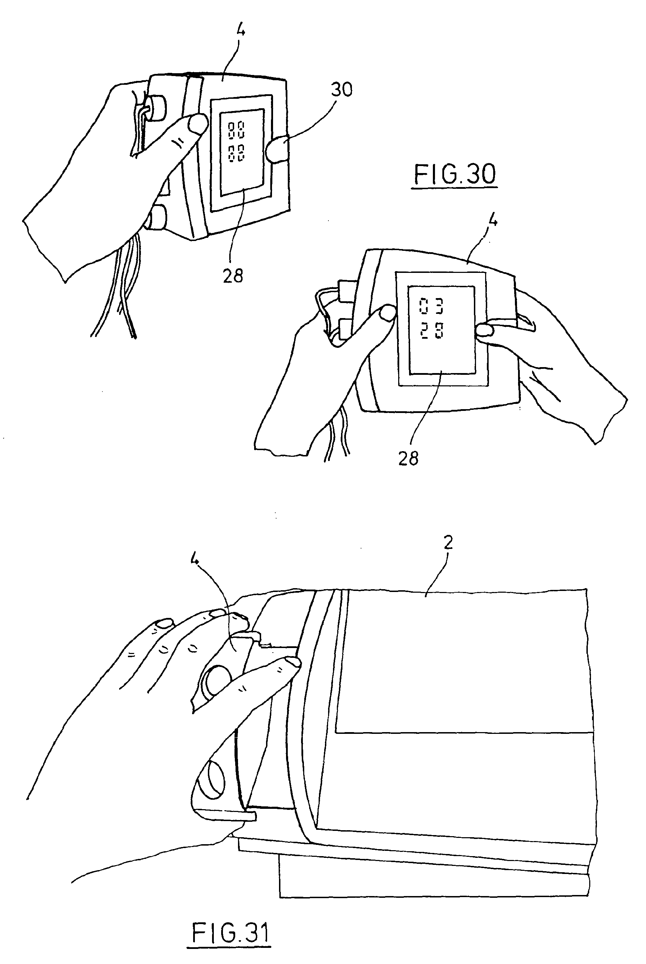

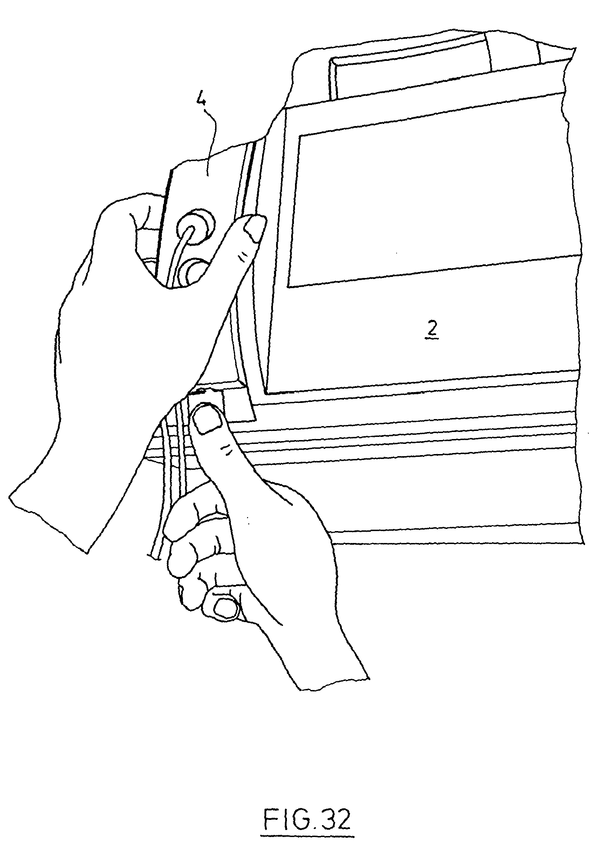

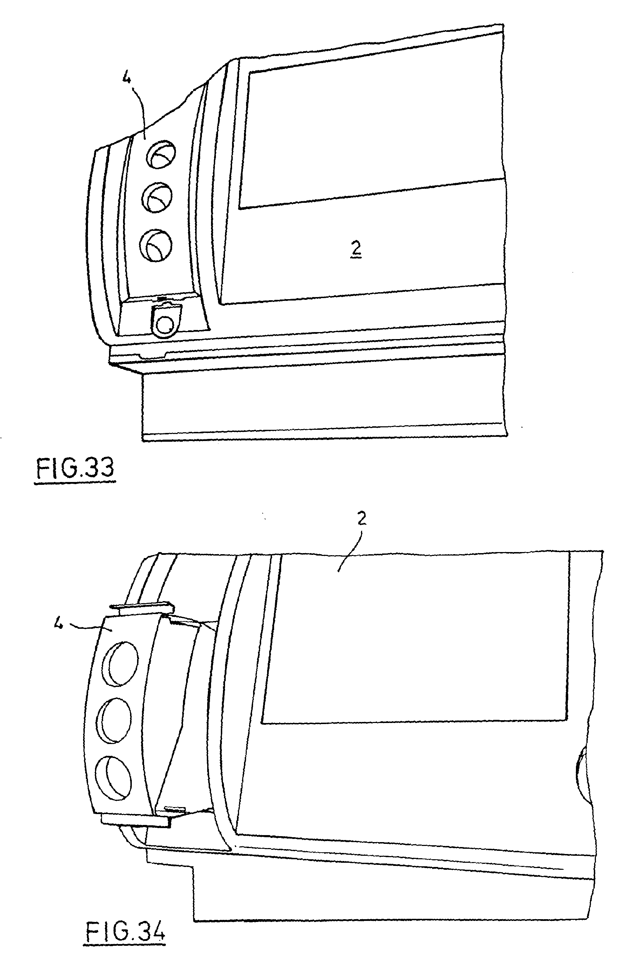

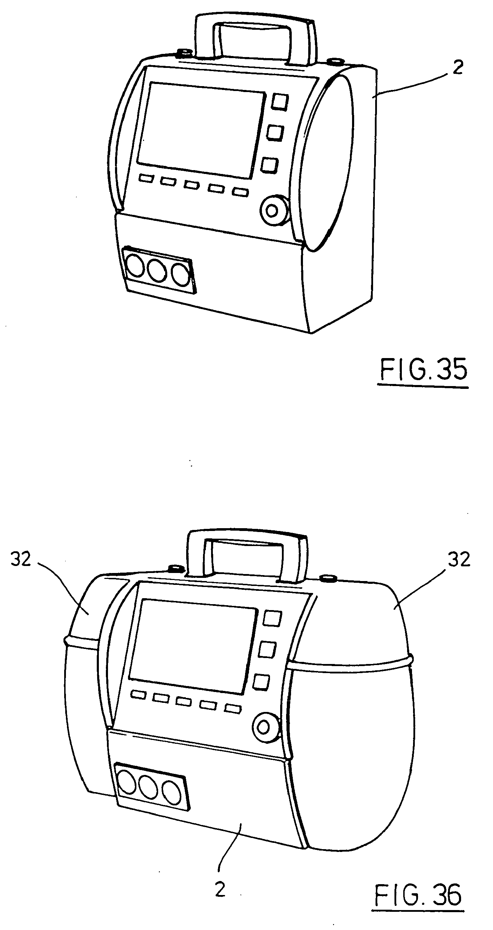

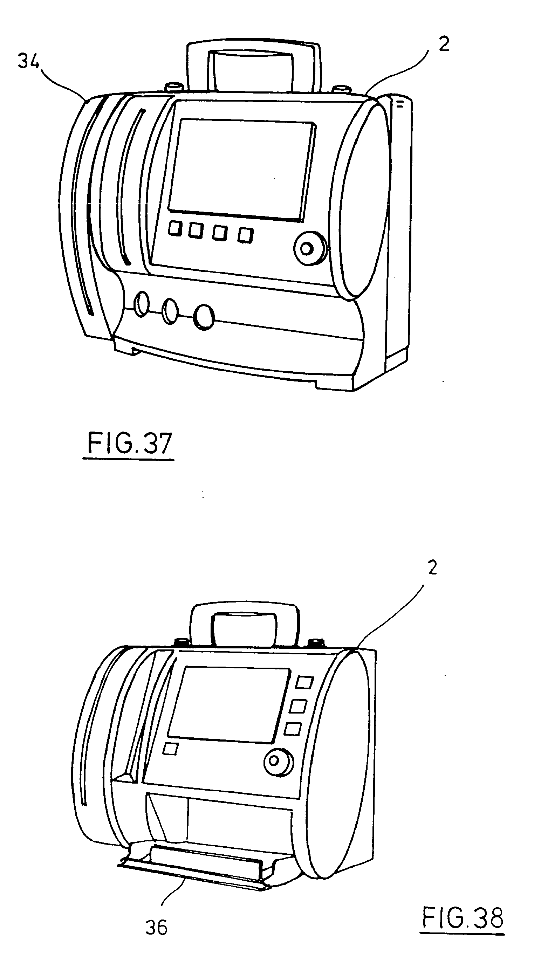

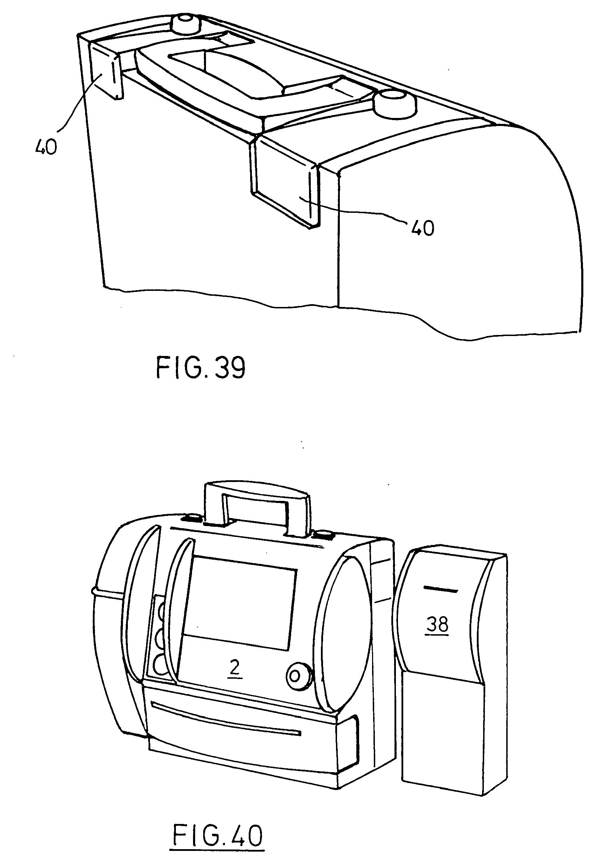

Die Fig. 25 bis 40 zeigen Ausführungsbeispiele eines tragbaren Basisgeräts (2), das mit Zusatzmodulen (32), seitlichen Taschen (34) sowie mit einer unten angeordneten Zubehörklappe (36) oder einem Drucker (38) versehen ist. Ferner ist das tragbare Basisgerät (2) an seiner Unterseite mit Haken (40) zur Befestigung an einer Wand oder einem Bett versehen.

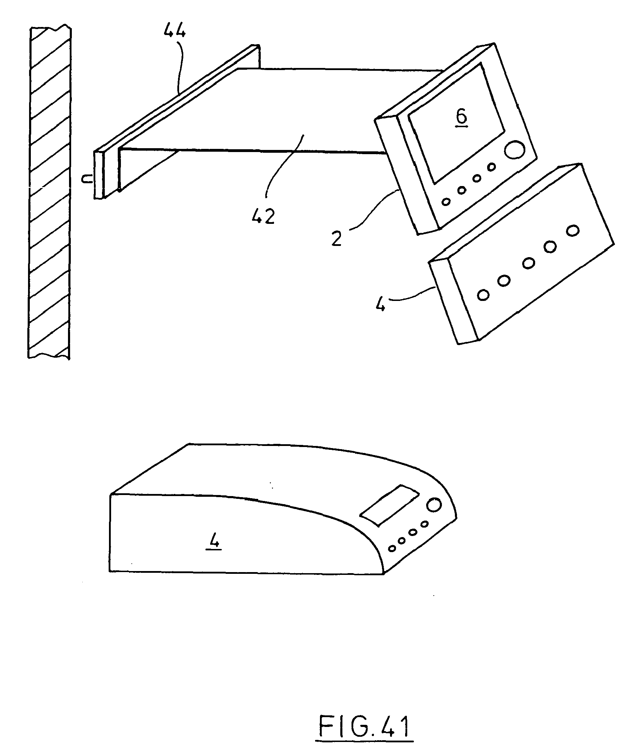

Ein weiteres Ausführungsbeispiel ist in Fig. 41 gezeigt.

Eine Konsole (42) ist mittels einer Wandschiene (44) an einer Wand befestigt. An der Konsole (42) ist ein Basisgerät (2) mit einem Monitor vorgesehen, der eine beispielsweise als Touchscreen ausgebildete Anzeige (8) sowie ein Modul (4) zum Vital- oder Zusatz-Monitoring umfasst.

Auf der Konsole (42) findet ein Therapiegerät (4) Platz, dass nur rudimentäre Anzeige- und Steuerelemente aufweist. Das Therapiegerät (4) wird über die Anzeige (6) des Basisgeräts (2) sowie über eine Kommunikationsverbindung zwischen dem Basisgerät (2) unter dem Therapiegerät (4) gesteuert. Dabei sind sowohl das Basisgerät (2) als auch das Therapiegerät (4) programmtechnisch derart eingerichtet, dass sie automatisch eine Kommunikationsverbindung aufbauen und wechselseitig Daten, wie z.B. Geräte- und Patientenkennungen sowie Patientenparameter und -daten austauschen.

Wenn ein Patient verlegt werden soll oder zu Untersuchungen oder Operationen transportiert werden muß, ist es lediglich erforderlich, das Therapiegerät (4) von der Konsole (42) abzunehmen und auf das Patientenbett zu legen. Da die Verbindung vom Basisgerät (2) zum Therapiegerät (4) drahtlos ist, ist es hierbei nicht erforderlich, Drahtverbindungen zu öffnen. Es verbleiben lediglich die Leitungen, die vom Therapiegerät (4) zum Patienten verlaufen. Grundsätzlich kann aber auch eine drahtgebundene Verbindung verwendet werden. Auch das Modul (4) kann abgenommen und transportiert werden, die Kabel zum Patienten verbleiben dabei am Gerät.

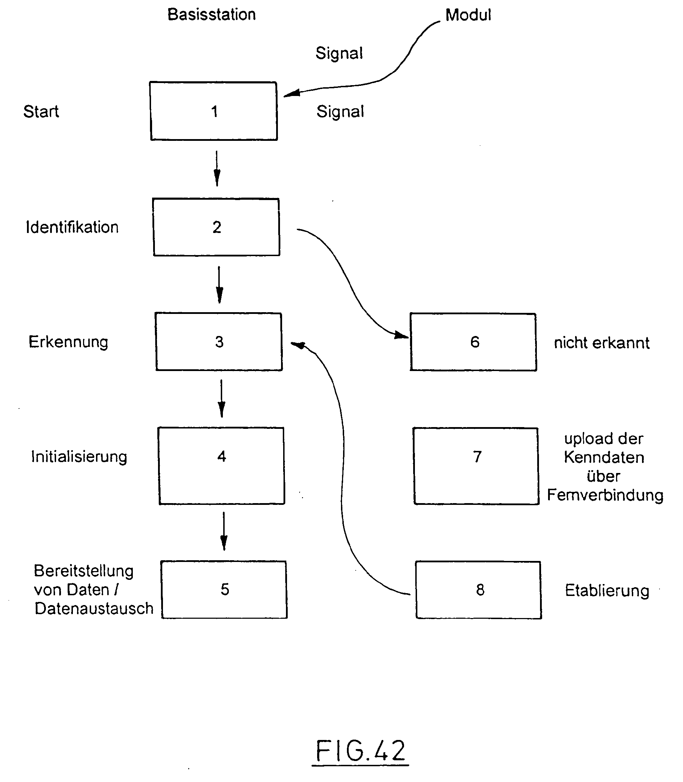

Nun wird anhand Fig. 42 der Vorgang des Aufbaus einer Kommunikationsverbindung zwischen einer Basisstation (2) und einem Modul (4) erläutert.

In den ersten beiden Schritten erfolgt nach Inbetriebnahme der Basisstation der Programmstart des in der Basisstationen abgespeicherten Programms sowie nach einem Signalaustausch mit den Modulen die Identifikation der Module, beispielsweise über RFID-Chips.

Wenn im dritten Schritt eine Erkennung erfolgt, folgt eine Initialisierung sowie die Bereitstellung von Daten beziehungsweise ein Datenaustausch mit dem erkannten Modul.

Wenn jedoch ein Modul nicht erkannt wird, wird seitens der programmtechnisch eingerichteten Basisstation eine Aktualisierung der Kenndaten über eine Fernverbindung veranlasst. Diese aktualisierten Kenndaten werden intern abgespeichert, um Zukunft einer weiteren Erkennung zur Verfügung zu stehen.

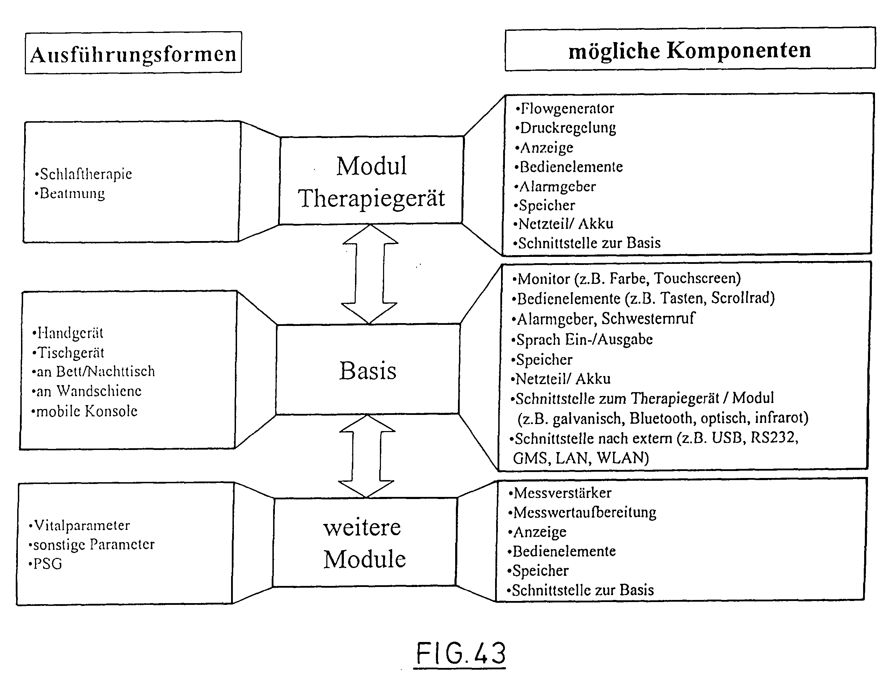

Fig. 43 veranschaulicht das modulare Gerätekonzept für verschiedene Anwendungen unter Einbeziehung diverser Funktionskomponenten.

Bei einer Realisierung des Funktionsmoduls im Zusammenhang mit einem modularen Defibrillator ist es möglich, das Funktionsmodul als tragbare Einheit auszubilden. Das Funktionsmodul übernimmt hierbei das Monitoring von Patientenparametern. Folgende Parameter können hierbei erfaßt werden: Sauerstoffsättigung des Blutes, Blutdruck, Pulsfrequenz sowie EKG. Bevorzugt werden mindestens zwei der vorstehend aufgeführten Parameter in Kombination miteinander erfaßt. Besonders bevorzugt mindestens das EKG-Signal im Zusammenhang mit mindestens einem der drei weiteren aufgeführten Parameter.

Gerätetechnisch ist es möglich, die Erfassung von mindestens einem der Patientenparameter unter Verwendung eines Zusatzmoduls durchzuführen, das an ein Grundgerät adaptierbar ist.

Der Defibrillator übt mindestens zwei der folgenden Funktionen aus: automatische Defibrillation, manuelle Defibrillation sowie Kardioversion. Unter Kardioversion wird hierbei eine synchronisierte Defibrillation verstanden, bei der ein Schock innerhalb einer definierten Zeit nach Erkennung der R-Zacke generiert wird. Vorzugsweise erfolgt eine manuelle Defibrillation in Kombination mit einer der beiden anderen Funktionen.

Durch die Aufteilung des Defibrillators in ein Basisgerät und ein Funktionsmodul läßt sich für das Funktionsmodul ein Gewicht von weniger als fünf Kilogramm erreichen, typischerweise fünf Kilogramm +/- 10 Prozent. Darüber hinaus läßt sich ein Volumen von weniger als 15 Liter erreichen, typischerweise 15 Liter +/- 10 Prozent.

Der Defibrillator kann mit einer Anzeige zur optischen Darstellung von Patientenparametern ausgestattet sein. Bevorzugt wird ein Display verwendet.

Die während einer Verwendung des Funktionsmoduls auftretenden Ereignisse werden komplett oder selektiert im Bereich des tragbaren Funktionsmoduls gespeichert und sind aus diesem auslesbar. In mindestens einem Betriebszustand kommuniziert die tragbare Einheit als Funktionsmodul mit der Basisstation. In mindestens einem Betriebzustand ermöglicht das Funktionsmodul als tragbare Einheit den Datenaustausch mit der Basisstation.

Das Funktionsmodul ist mit einer autonomen Energieversorgung versehen, beispielsweise einem Akkumulator oder einer Batterie. Typischerweise reicht der verfügbare Energieinhalt aus, um wenigstens die wesentlichen Funktionen für mindestens 30 Minuten durchführen zu können. Der Energieinhalt ist ebenfalls dafür ausreichend, innerhalb von 30 Minuten mindestens zwei Schocks abgeben zu können.

Für eine wiederaufladbare Energieversorgung des Funktionsmoduls ist vorgesehen, eine Wiederaufladung im Bereich der Basisstation durchzuführen. Es können aber auch separate Ladegeräte verwendet werden.

Hinsichtlich des Gewichts des Funktionsmoduls besteht ein Gesamtgewicht des Komplettgerätes von Einrichtungen gemäß dem Stand der Technik von etwa 6 Kilogramm. Durch die vorgeschlagene Trennung des Gerätes in das Funktionsmodul und das Basisgerät ist es möglich, einen Drucker, einen Patientenkartenleser, ein GSM-Modul sowie eine Schnittstelle für externe Speichermedien im Bereich der Basisstation anzuordnen. Diese Bauelemente mit einem Gesamtgewicht von etwa einem Kilogramm brauchen somit nicht im Bereich des Funktionsmoduls angeordnet zu werden.

Ein Gesamtgerät gemäß dem Stand der Technik weist ein Volumen von etwa 20 Litern auf. Die Anordnung der oben aufgeführten Bauelemente im Bereich des Basismoduls führt zu einer Volumenreduktion im Bereich des Funktionsmoduls von etwa 2,5 bis 5 Liter.

Hinsichtlich der Konstruktion eines modularen Beatmungsgerätes kann gemäß einer bevorzugten Ausführungsform das Volumen des tragbaren Funktionsmoduls um etwa 15 % gegenüber einem Gerät nach dem Stand der Technik vermindert werden.

Ebenfalls lassen sich im Vergleich mit einem Gerät nach dem Stand der Technik die Herstellungskosten um etwa 15 % reduzieren.

Ein tragbares Funktionsmodul zur Verwendung als Beatmungsgerät für die invasive und/oder nicht invasive Beatmung besitzt typischerweise ein Gerätevolumen von weniger als drei Liter. Bevorzugt ist ein Volumen in einem Bereich von drei Liter +/- 10 Prozent hinsichtlich eines nicht invasiven Beatmungsgerätes. Bezüglich eines invasiven Beatmungsgerätes beträgt ein typisches Volumen weniger als vier Liter, bevorzugt vier Liter +/- 10 Prozent. Das Funktionsmodul kann mit einem Flowsensor und/oder einem Drucksensor ausgestattet sein und über eine elektronisch gesteuerte Druckgasquelle verfügen. Ein typischer Druckbereich liegt zwischen 0 und 80 mbar. Die Energieversorgung kann kabelgebunden und/oder kabellos erfolgen. Neben den Beatmungsmodi CPAP und BiLevel können auch weitere Beatmungsmodi realisiert werden.

zur Anzeige von gemessenen Parametern, Bedienhinweisen oder Statussignalen kann eine Anzeigeeinrichtung verwendet werden, beispielsweise ein Display. Bevorzugt erfolgt eine optische Anzeige eines Betriebszustandes.

Das tragbare Funktionsmodul kommuniziert in mindestens einem Betriebszustand mit einer Basisstation und/oder ermöglicht in mindestens einem Betriebszustand den Datenaustausch mit der Basisstation.

Gemäß einem Ausführungsbeispiel besitzt das tragbare Funktionsmodul einen internen Datenspeicher mit einer Speicherkapazität von mehr als 100 kb.

Hinsichtlich der Datenerfassung können in mindestens einem Betriebsmodus mindestens zwei der nachfolgenden Ereignisse in einem Zeitbereich von weniger als einer Minute erfaßt werden: Zunahme des Atemflusses, Abnahme des Atemflusses, Abflachung des Atemflusses, Stillstand des Atemflusses, Zunahme der Resistance, Veränderung einer Leckage, Einatmung, Ausatmung, Atempause, Zunahme des Atemvolumens, Abnahme des Atemvolumens, Zunahme des Druckes des Atemfrischgases, Abnahme des Druckes des Atemfrischgases, Zunahme des geförderten Volumens an Atemfrischgas sowie Abnahme des geförderten Volumens an Atemfrischgas.

## Patentansprüche

1. Medizintechnisches Gerät zum Monitoring und/oder zur Therapie, mit einer Basisstation (2) und wenigstens einem Modul (4), **dadurch gekennzeichnet, dass** das wenigstens eine Modul (4) im von der Basisstation (2) getrennten Zustand funktionsfähig ist.

2. Medizintechnisches Gerät nach Anspruch 1, **dadurch gekennzeichnet, dass** die Basisstation (2) wenigstens eine Anzeigeeinrichtung (6) aufweist.

3. Medizintechnisches Gerät nach Anspruch 1 oder 2, **dadurch gekennzeichnet, dass** die Basisstation (2) zum Datenaustausch mit wenigstens einem Modul (4) ausgebildet ist.

4. Medizintechnisches Gerät nach Anspruch 3, **dadurch gekennzeichnet, dass** die Basisstation (2) zum drahtlosen und/oder drahtgebundenen Datenaustausch ausgebildet ist.

5. Medizintechnisches Gerät nach einem der vorherigen Ansprüche, **dadurch gekennzeichnet, dass** die Basisstation (2) eine Aufnahme für wenigstens ein Modul (4) aufweist.

6. Medizintechnisches Gerät nach einem der vorherigen Ansprüche, **dadurch gekennzeichnet, dass** die Basisstation (2) und das wenigstens eine Modul (4) komplementär zueinander ausgebildete Ladekontakte aufweisen.

7. Medizintechnisches Gerät nach einem der vorherigen Ansprüche, **dadurch gekennzeichnet, dass** die Basisstation (2) eine Alarmeinrichtung aufweist.

8. Medizintechnisches Gerät nach einem der vorherigen Ansprüche, **dadurch gekennzeichnet, dass** die Basisstation (2) tragbar ausgebildet ist.

9. Medizintechnisches Gerät nach einem der vorherigen Ansprüche, **dadurch gekennzeichnet, dass** die Basisstation (2) eine Einrichtung zur netzunabhängigen Energieversorgung aufweist.

10. Medizintechnisches Gerät nach einem der vorherigen Ansprüche, **dadurch gekennzeichnet, dass** das Modul (4) eine Einrichtung zur netzunabhängigen Energieversorgung aufweist.

11. Medizintechnisches Gerät nach einem der vorherigen Ansprüche, **dadurch gekennzeichnet, dass** das Modul (4) als Therapiegerät, vorzugsweise als Beatmungsgerät, als Absaugpumpe, als Sauerstoffgerät, als Monitoringmodul oder als Defibrillator ausgebildet ist.

12. Medizintechnisches Gerät, weiter umfassend eine Dockingstation (8) zur Aufnahme einer Basisstation (2).

13. Medizintechnisches Gerät nach Anspruch 11, **dadurch gekennzeichnet, dass** die Dockingstation (8) und das Basisgerät (2) komplementär zueinander ausgebildete Ladekontakte aufweisen.

14. Basisstation (2) nach einem der vorherigen Ansprüche.

15. Modul (4) nach einem der vorherigen Ansprüche.

16. Docking-Station (8) nach einem der vorherigen Ansprüche.

17. Verfahren zur Steuerung eines medizintechnischen Gerätes, bestehend aus einer Basisstation und mindestens einem adaptierten Modul, **dadurch gekennzeichnet, daß** bei einer Annäherung des Moduls an die Basisstation eine automatische Erkennung und in zumindest einem Betriebszustand ein Datenaustausch erfolgt.

18. Medizintechnisches Gerät, das eine Basisstation und mindestens ein Funktionsmodul aufweist, **dadurch gekennzeichnet, daß** eine Ausbildung als modularer Defibrillator realisiert ist.

19. Medizintechnisches Gerät nach Anspruch 18, **dadurch gekennzeichnet, daß** der Defibrillator mindestens zwei Funktionszustände aufweist, nämlich eine manuelle Defibrillation und zusätzlich eine automatische Defibrillation oder eine Kardioversion und daß das Funktionsmodul als tragbare Einheit einen Speicher aufweist und zur mindestens zeitweisen Kommunikation mit der Basisstation ausgebildet ist.

20. Medizintechnisches Gerät, das eine Basisstation und mindestens ein Funktionsmodul aufweist, **dadurch gekennzeichnet, daß** eine Ausbildung als modulares Beatmungsgerät realisiert ist.

21. Medizintechnisches Gerät nach Anspruch 20, **dadurch gekennzeichnet, daß** das modulare Beatmungsgerät zur Durchführung einer invasiven Beatmung ausgebildet ist.

22. Medizintechnisches Gerät nach Anspruch 20, **dadurch gekennzeichnet, daß** das modulare Beatmungsgerät zur Durchführung einer nicht invasiven Beatmung ausgebildet ist.

23. Medizintechnisches Gerät, das eine Basisstation und mindestens ein Funktionsmodul aufweist, **dadurch gekennzeichnet, daß** das Funktionsmodul mit einem Flowsensor und/oder einem Drucksensor versehen ist, daß eine Druckgasquelle des Funktionsmoduls eine elektronische Steuerung aufweist und daß das Funktionsmodul zur wenigstens zeitweisen Kommunikation mit der Basisstation ausgebildet ist.
